(19) Europäisches Patentamt

European Patent Office

Office européen des brevets

(11) **EP 1 264 894 A1**

(12) **EUROPEAN PATENT APPLICATION**
published in accordance with Art. 158(3) EPC

(43) Date of publication:
**11.12.2002 Bulletin 2002/50**

(51) Int Cl.7: **C12P 19/32**, C12Q 1/48,
C12Q 1/66, C12Q 1/68

(21) Application number: **01901364.8**

(22) Date of filing: **17.01.2001**

(86) International application number:
**PCT/JP01/00238**

(87) International publication number:
**WO 01/053513 (26.07.2001 Gazette 2001/30)**

(84) Designated Contracting States:
**AT BE CH CY DE DK ES FI FR GB GR IE IT LI LU MC NL PT SE TR**

(30) Priority: **17.01.2000 JP 2000007332**
**07.02.2000 JP 2000028976**
**14.04.2000 JP 2000112790**
**20.04.2000 JP 2000119798**
**29.11.2000 JP 2000362340**

(71) Applicant: **Satake Corporation**
**Chiyoda-ku, Tokyo 101-0021 (JP)**

(72) Inventors:
• **OHTAKE, Hisao**
**Higashihiroshima-Shi, Hiroshima 739-0023 (JP)**
• **KURODA, Akio**
**Higashihiroshima-Shi, Hiroshima 739-0045 (JP)**
• **TANAKA, Shotaro**
**Higashihiroshima-Shi, Hiroshima 739-0041 (JP)**

(74) Representative: **Bates, Philip Ian**
**Reddie & Grose**
**16 Theobalds Road**
**London WC1X 8PL (GB)**

(54) **ATP REGENERATION REACTION SYSTEMS AND METHOD OF EXAMINING ADENINE NUCLEOTIDE, METHOD OF DETECTING RNA AND METHOD OF AMPLIFYING ATP BY USING THE**

(57)     The present invention relates to an ATP-regeneration reaction system comprising the steps of acting adenylate kinase on AMP to convert it into ADP and acting a polyphosphoric acid synthetase in the presence of a polyphosphoric acid compound to convert it into ATP and a polyphosphoric acid compound; an ATP-regeneration reaction system comprising the steps of acting a phosphotransferase on AMP in the presence of a polyphosphoric acid compound to convert it into ADP and then acting a polyphosphoric acid synthetase on the resulting ADP to convert it into ATP; and a method for detecting or inspecting adenine nucleotide or RNA and an ATP-amplification method, which make use of the foregoing regeneration system.

FIG.1

# EP 1 264 894 A1

**Description**

Technical Field

The present invention relates to an ATP regeneration reaction system, and a method for examining adenine nucleotide, a method for detecting RNA and a method for amplifying ATP, using the reaction system.

More specifically, the present invention pertains to a method for examining so-called adenine nucleotides such as ATP, ADP, AMP and mixture thereof, which are generated in the metabolic and/or biosynthetic systems of animals and vegetables. The present invention can be applied to, for instance, the examination of the cleanliness factor by the detection of invisible microorganisms present in, for instance, food factories and the determination of the degree of freshness of foods such as meat, raw fishes and vegetables, by the determination of ATP (adenosine triphosphate) through, for instance, bioluminescence.

Moreover, the present invention also relates to a method for detecting ribonucleic acid (RNA), in particular, messenger RNA (mRNA), ribosomal RNA (rRNA) and transfer RNA (tRNA), which are present in the cells of higher animals and closely related to the protein synthesis.

In addition, the present invention likewise relates to a method for amplifying the amount of ATP generated, in a manner like a chain reaction, in the metabolic and/or biosynthetic systems of animals and vegetables. A trace amount of ATP, which has not been able to be detected by any conventional technique, can be detected by the detection of bioluminescence using this method and accordingly, the method may be applicable to the examination of the cleanliness factor by the detection of invisible microorganisms present in, for instance, food factories and the determination of the degree of freshness of foods such as meat, raw fishes and vegetables.

Background Art

ATP (adenosine triphosphate) may serve as an indication of the living organisms. Therefore, there have been conducted sanitary examinations of microorganisms while using light rays as an indication by the use of ATP derived from microorganisms in the bioluminescence technique (see, for instance, Japanese Examined Patent Publication (hereunder referred to as "J.P. KOKOKU") No. Hei 6-34757, Japanese Patent No. 1,911,659). When the amount of microorganisms is very small, however, the bioluminescence derived from ATP is insufficient.

For instance, it has been known that luminescent rays are emitted, if acting an enzyme (such as luciferase derived from a lightening bug) serving as a catalyst on ATP as an indicative component of bacterial contamination. However, the foregoing determination of the emitted luminescent rays suffers from a problem in that the stability of the light emission is low and that the emitted luminescent rays disappear within a very short period of time. Accordingly, the method requires strict control of the reaction time and the use of a specially designed luminometer for picking up the emitted light rays, which disappear within a short period of time, in order to ensure a desired sensitivity and precision.

To solve the foregoing problems, Japanese Un-Examined Patent Publication (hereunder referred to as "J.P. KOKAI") No. Hei 8-47399 discloses a method for determining bioluminescence generated by luciferase, wherein the bioluminescent reaction is conducted in the co-existence of a polyphosphoric acid compound or a salt thereof and a sulfhydryl compound. This method permits the achievement of a novel effect or the stabilization of bioluminescence and it in turn permits the determination of such a bioluminescent reaction using a currently used luminometer and increases the rate of propagation of this method. The term "polyphosphoric acid compound" used in this patent (J.P. KOKAI No. Hei 8-47399) means triphosphoric acids, diphosphoric acids or salts thereof such as tripolyphosphoric acid and pyrophosphoric acid.

However, the method disclosed in the foregoing patent, J.P. KOKAI No. Hei 8-47399, suffers from such a problem that the reaction system does not include any fresh ATP-regeneration system and therefore, the intensity of the emitted light rays is gradually attenuated with time as the ATP is consumed. For this reason, it would be obliged to study substrate concentration, oxygen concentration, pH value and temperature in order to stably maintain the quantity of the emitted light without causing any attenuation thereof.

On the other hand, J.P. KOKAI No. Hei 9-234099 discloses a method for quantitatively determining the amount of cyclic AMP by means of the bioluminescence technique.

This method is characterized in that it comprises "a reaction 1" wherein the cyclic AMP is hydrolyzed with cyclic 3',5'-nucleotide phosphodiesterase to thus generate AMP in the reaction system; "a reaction 2" wherein adenylate kinase is acted on the AMP in the presence of magnesium ions and a trace amount of ATP to convert the AMP into ADP; "a reaction 3" wherein pyruvate kinase is acted on the resulting ADP in the presence of magnesium ions and phosphoenol pyruvic acid to thus convert them into ATP and pyruvic acid; "a reaction 4" wherein luciferase is acted on the resulting ATP in the presence of luciferin, magnesium ions (or ions of other metals) and dissolved oxygen to thus induce light emission; and a step of detecting the intensity of the light emitted in the "reaction 4" to thus quantitatively determine the amount of the cyclic AMP (Method in Enzymology, 1974, 38: 62-65).

In this method, however, AMP converted from the cyclic AMP is converted into ADP and is further converted into ATP by acting pyruvate kinase on the ADP in the presence of phosphoenol pyruvic acid and therefore, the method suffers from the following problems. The phosphoenol pyruvic acid includes only one phosphate residue ($-PO_3^-$-) in the molecule and this makes the conversion of ADP into ATP insufficient. For this reason, a large quantity of phosphoenol pyruvic acid should be supplemented to the reaction system to regenerate a large quantity of ATP and therefore, this method is unfavorable from the economical standpoint.

Moreover, phosphoenol pyruvic acid is quite susceptible to heat and easily undergoes decomposition. For this reason, if a subject to be examined is cooked rice immediately after the cooking in a rice-cooking factory and a reagent including phosphoenol pyruvic acid is administered thereto, the phosphoenol pyruvic acid is decomposed and this sometimes makes the conversion of ADP into ATP insufficient (in this respect, however, a heat-resistant enzyme may be used when the subject to be examined is maintained at a temperature ranging from about 60 to 70°C). More specifically, the luminescent emission due to the presence of ATP is unstable and the emission may disappear within a very short period of time.

Moreover, each subject to be examined in general includes several kinds of bacterial cells and if certain bacterial cells include an enzyme capable of decomposing phosphoenol pyruvic acid, any phosphoric acid is not supplemented and therefore, the conversion of ADP into ATP may become insufficient.

Incidentally, J.P. KOKOKU No. Sho 53-5752 and Domestic Re-Publication of PCT International Publication (hereunder referred to as "Re-Publication") No. WO98/48031 disclose a method for economically preparing ATP while making use of an ATP-regeneration reaction system. More specifically, the method disclosed in J.P. KOKOKU No. Sho 53-5752 is characterized by acting a polyphosphoric acid-synthesizing enzyme and adenylate kinase on AMP, ADP and a polyphosphoric acid compound. This method comprises the steps of acting the polyphosphoric acid-synthesizing enzyme on one molecule of ADP and the polyphosphoric acid compound to give one molecule of ATP; acting the adenylate kinase on the resulting one molecule of ATP and one molecule of AMP to give two molecules of ADP; and again acting the polyphosphoric acid-synthesizing enzyme on the two molecules of ADP and the polyphosphoric acid compound to thus give two molecules of ATP. However, this method intends to synthesize a large quantity of ATP from AMP and therefore, the resulting ATP may contain a large amount of ADP. Accordingly, this method cannot be applied to the examination of cleanliness factor and the determination of the degree of freshness of foods, through the detection of the presence of ATP in a trace amount.

In addition, the method disclosed in Re-Publication WO98/48031 is characterized in that it comprises the step of acting a polyphosphoric acid-synthesizing enzyme and adenylate kinase on AMP and a polyphosphoric acid compound. This method accordingly permits the preparation of ATP from cheap AMP without using any expensive ATP, or the efficient ATP-regeneration from the consumed ATP, in the enzyme reaction system, which makes use of ATP. However, it is recognized that this ATP-preparation method permits the preparation of ATP from cheap AMP even in the absence of any ATP at the initial stage of the reaction. Therefore, this method cannot be applied to the examination of cleanliness factor and the determination of the degree of freshness of foods, through the detection of the presence of ATP in a trace amount.

Furthermore, it has been known that the ribonucleic acid (RNA) present in the cells of higher organisms and involved in the protein synthesis plays an intermediary role or such a role that it transfers the genetic information of a DNA to other cells (HIROUMI Keitaro, "Biochemistry Based on Experiments", p. 205, 1987/7/10, published by KAGAKU DOJIN Publishing Company). It has been proved that the sequence of a protein synthesized in the cytoplasm is determined by the order of the base sequence of the DNA present in the nucleus. In the genetic code (codon), a combination of 3 different bases free of any overlapping known as the coding triplet defines each specific amino acid. The genetic information is transferred by an intermediary molecule referred to as the messenger RNA (mRNA) from the nucleus to a protein-synthesizing system on a ribosome. This molecule has a base sequence complementary to that of the DNA of a nucleus, the combination of 3 bases on the mRNA corresponding to a coding triplet is known as a codon and in a first stage, the DNA serves as a "template" for synthesizing a corresponding mRNA.

The mRNA is transported to a ribosome and takes part in the synthesis of a specific protein with the assistance of a tRNA. If the composition of such an RNA molecule is clarified, it would widely be applied to, for instance, fields relating to the health of human beings (for instance, pathologic diagnosis and development of therapeutic agents) and fields in which foods are inspected for any contamination with bacteria (such as the examination of the cleanliness factors thereof) and it has attracted special interest in the recent fields concerning, for instance, metabolic and/or biosynthesis systems.

The technique for determining the composition of a specific RNA molecule is well known. For instance, one of well known methods comprises the steps of isolating RNA molecules from yeast cells by the centrifugation and determining absorption spectra using a ultraviolet spectrophotometer (chromatography); or comprises the steps of hydrolyzing RNA molecules, separating the RNA fragments (nucleotides) obtained by the hydrolysis according to the electrophoresis and then determining absorption spectra of the nucleotides.

In addition, J.P. KOKOKU No. Hei 8-2320 discloses a method for detecting a polynucleotide carrying a 3'-terminal polyriboadenosine segment in a sample. This method comprises the following steps: (a) a step of digesting nucleic acids present in a sample with polynucleotide phosphorylase in the presence of an inorganic phosphate to convert the 3'-terminal polyriboadenosine segment into ADP-containing ribonucleoside diphosphate; (b) a step of phosphorylating the ADP generated in the digestion step (a) to give ATP; and (c) a step of detecting either the ATP molecules or by-products obtained in the phosphorylation step (b). This prior art also discloses that the polynucleotide carrying a 3'-terminal polyriboadenosine segment is mRNA derived from a eucaryotic organism and likewise discloses a technique, used in the ATP detection step, which comprises the steps of reacting ATP with luciferin in the presence of luciferase to thus induce a bioluminescent reaction and then determining the amount of the ATP.

Thus, the composition of the mRNA derived from a eucaryotic organism can be determined by the bioluminescence due to ATP and the digestion of mRNA into AMP or ADP and the phosphorylation of the ADP into ATP would permit the quantitative determination of each component of the mRNA.

As has been discussed above, J.P. KOKOKU No. Hei 8-2320 discloses a technique for determining the composition of the mRNA of the eucaryotic organism by the bioluminescence through the enzyme reaction of ATP. However, the determination based on the bioluminescence disclosed in this patent suffers from a problem in that the stability of the light emission is low and that the emitted light rays disappear within a very short period of time. Accordingly, the method requires the strict control of the reaction time and the use of a specially designed luminometer for picking up the emitted light rays, which disappear within a short period of time, in order to ensure a desired sensitivity and precision.

J.P. KOKAI No. Hei 11-69997 discloses an inspection agent for examining the cleanliness factor of a sample capable of detecting, with a high sensitivity, ADP, AMP and RNA which have not been easily detected or determined by the conventional luciferin-luciferase luminescent reagent; simultaneous with ATP serving as a component indicative of bacterial contamination of the sample even at a low degree of contamination and also capable of accurately evaluating the cleanliness factor, as well as a method for examining the cleanliness factor of a sample using the foregoing inspection agent. The determination of RNA according to this method comprises the steps of acting an RNase on RNA to form 5'-mononucleotides (AMP, GMP, CMP, UMP), converting the AMP out of the resulting mononucleotides into ATP, acting luciferase on the resulting ATP in the presence of luciferin to thus induce light emission and determining the quantity of the emitted light to thus evaluate the amount of RNA present in the sample. In this reaction system, AMP and pyrophosphoric acid are formed in the final step thereof and ATP is regenerated from these substances. Therefore, it has been recognized that the luminescence generated in a series of ATP-conversion reaction systems never undergoes any attenuation and it is stably sustained over at least 10 minutes at a high level. The ATP-regeneration system comprises the step of acting pyruvate orthophosphate dikinase on AMP, pyrophosphoric acid and phosphoenol pyruvic acid. As has been described above, however, the phosphoenol pyruvic acid includes only one phosphate residue in the molecule and this makes the conversion of AMP into ATP insufficient. For this reason, a large quantity of phosphoenol pyruvic acid should be supplemented to the reaction system to regenerate a large quantity of ATP and therefore, this method is unfavorable from the economical standpoint. Moreover, phosphoenol pyruvic acid is quite susceptible to heat and easily undergoes decomposition. For this reason, if the phosphoenol pyruvic acid is added to a subject to be examined maintained at a high temperature, it is easily decomposed and this sometimes makes the conversion of AMP into ATP insufficient. More specifically, the luminescent emission due to ATP is unstable and the emission may disappear within a very short period of time.

Disclosure of the Invention

Accordingly, it is a first object of the present invention to provide a novel ATP-regeneration system.

It is a second object of the present invention to provide a method for examining adenine nucleotide.

It is a third object of the present invention to provide a method for detecting RNA

It is a fourth object of the present invention to provide a method for amplifying ATP.

The present invention thus relates to an ATP-regeneration reaction system, and a method for examining adenine nucleotide, a method for detecting RNA and a method for amplifying ATP, which make use of the foregoing ATP-regeneration reaction system, as will be detailed below.

(1) An ATP-regeneration reaction system characterized in that it comprises the steps of acting adenylate kinase on AMP in the presence of a trace amount of ATP to convert them into two ADP molecules and then acting a polyphosphoric acid-synthesizing enzyme (polyphosphoric acid synthetase) on the resulting two ADP molecules in the presence of a polyphosphoric acid compound (n) (wherein n means the number of phosphate residues present in the polyphosphoric acid compound) to convert the two ADP molecules into two ATP molecules and a molecule of polyphosphoric acid compound (n-2).

(2) An ATP-regeneration reaction system characterized in that it comprises the steps of acting a phosphotransferase on AMP in the presence of a polyphosphoric acid compound (n) (wherein n means the number of phosphate residues present therein) to convert them into ADP and a polyphosphoric acid compound (n-1) and then acting a polyphosphoric acid synthetase on the ADP in the presence of the polyphosphoric acid compound (n-1) to thus convert the ADP into ATP and a polyphosphoric acid compound (n-2).

(3) A method for detecting or inspecting adenine nucleotide according to the bioluminescence, which comprises the steps of acting luciferase on ATP in the presence of luciferin and dissolved oxygen to thus generate AMP and to induce light emission and determining the quantity of emitted light, wherein the method is provided with an ATP-regeneration reaction system characterized in that it comprises the steps of acting adenylate kinase on AMP in the presence of a trace amount of ATP to convert them into two ADP molecules and then acting a polyphosphoric acid synthetase on the resulting two ADP molecules in the presence of a polyphosphoric acid compound (n) (wherein n means the number of phosphate residues present therein) to convert them into two ATP molecules and a molecule of polyphosphoric acid compound (n-2) and that the trace amount of ATP is one derived from contamination.

(4) A method for detecting or inspecting adenine nucleotide according to the bioluminescence, which comprises the steps of acting luciferase on ATP in the presence of luciferin and dissolved oxygen to thus generate AMP and induce light emission and determining the quantity of emitted light, wherein the method is provided with an ATP-regeneration reaction system characterized in that it comprises the steps of acting a phosphotransferase on AMP in the presence of a polyphosphoric acid compound (n) (wherein n means the number of phosphate residues present therein) to convert them into ADP and a polyphosphoric acid compound (n-1) and then acting a polyphosphoric acid synthetase on the ADP in the presence of the polyphosphoric acid compound (n-1) to thus convert them into ATP and a polyphosphoric acid compound (n-2).

(5) A method for detecting or inspecting RNA according to the bioluminescence, which comprises the steps of treating RNA present in a sample with a ribonuclease to give mononucleotides, converting the AMP in the mononucleotides into ATP, acting luciferase on the ATP in the presence of luciferin and dissolved oxygen to induce light emission, and determining the quantity of emitted light to thus quantitatively determine the amount of the AMP present in the RNA, wherein the method is provided with an ATP-regeneration reaction system characterized in that it comprises the steps of acting a phosphotransferase on AMP in the presence of a polyphosphoric acid compound (n) (wherein n means the number of phosphate residues present therein) to convert them into ADP and a polyphosphoric acid compound (n-1) and then acting a polyphosphoric acid synthetase on the ADP in the presence of the polyphosphoric acid compound (n-1) to thus convert them into ATP and a polyphosphoric acid compound (n-2).

(6) A method for amplifying ATP, like a chain reaction, characterized in that the method comprises repeatedly conducting the following two steps: acting adenylate kinase on AMP in the presence of a trace amount of ATP to convert them into two ADP molecules and then acting a polyphosphoric acid synthetase on the resulting two ADP molecules in the presence of a polyphosphoric acid compound (n) (wherein n means the number of phosphate residues present therein) to convert them into two ATP molecules and a molecule of polyphosphoric acid compound (n-2).

(7) A method for detecting or inspecting adenine nucleotide, which comprises the steps of acting luciferase on ATP in the presence of luciferin and dissolved oxygen to thus generate AMP and induce light emission and determining the quantity of emitted light, wherein the method comprises the steps of repeatedly conducting the following two steps: acting adenylate kinase on AMP in the presence of the ATP present in a subject to be tested to convert them into two ADP molecules and then acting a polyphosphoric acid synthetase on the resulting two ADP molecules in the presence of a polyphosphoric acid compound (n) (wherein n means the number of phosphate residues present therein) to convert them into two ATP molecules and a molecule of polyphosphoric acid compound (n-2) to thus amplify ATP in a manner like a chain reaction and then acting luciferase on the amplified ATP in the presence of luciferin and dissolved oxygen to thus give AMP and to induce light emission.

Brief Description of the Drawings

Fig. 1 is a graph showing the changes with time of the relative amounts of emitted light observed for ATP-regeneration reaction systems of the present invention (a first embodiment) and the conventional technique, in which the ATP concentration is set at 1.65 $\mu$M.
Fig. 2 is a graph showing the results obtained by examining the changes of ATP concentration with time in a second embodiment of the ATP-regeneration reaction system according to the present invention.

Fig. 3 is a graph showing the changes with time of the relative amounts of emitted light observed when the second ATP-regeneration reaction system of the present invention is used in the determination of adenine nucleotide and when the system is not used in the determination.

Fig. 4 is a graph showing the results obtained when RNA is detected according to the bioluminescence using the second ATP-regeneration reaction system of the present invention.

Fig. 5 is a graph showing the results obtained by examining any influence of ATP (presence of added ATP: +ATP; free of any added ATP: -ATP) on the changes of the luminescence with time using a purified polyphosphoric acid synthetase (PPK [1]; Fig. 6(a)) and a polyphosphoric acid synthetase from which ADP has been removed (PPK [2]; Fig. 6(b)).

Fig. 6 is a graph showing the results obtained by examining any influence of ATP (presence of added ATP: +ATP; free of any added ATP: -ATP) on the changes of the luminescence with time in the ATP-regeneration reaction system in which the concentrations of polyphosphoric acid and AMP are changed. The concentrations of polyphosphoric acid and AMP are 900μM and 600 μM (a); 900 μM and 60 μM (b); 90 μM and 600 μM (c), respectively.

Fig. 7 is a graph showing changes, with time, in ATP concentration generated by the ATP-amplification reaction of the present invention, like a chain reaction.

Best Mode for Carrying Out the Invention

First of all, the first ATP-regeneration reaction system of the present invention will hereunder be described in detail.

The first ATP-regeneration reaction system of the present invention is one characterized by acting adenylate kinase on AMP in the presence of a trace amount of ATP to convert them into two ADP molecules and then acting a polyphosphoric acid synthetase on the resulting two ADP molecules in the presence of a polyphosphoric acid compound (n) (wherein n means the number of phosphate residues present therein) to convert them into two ATP molecules and a molecule of a polyphosphoric acid compound (n-2).

The polyphosphoric acid compound (n) (wherein n means the number of phosphate residues present therein) usable in the present invention is preferably one having a value of n ranging from 10 to 1000. Examples of such polyphosphoric acid compounds are those chemically synthesized and having 10 to 100 phosphate residues linearly linked together. The use of such a polyphosphoric acid compound would make the conversion of ADP into ATP easy and this in turn permits the reduction of the amount of the compound to be supplemented to the reaction system upon the regeneration of ATP and makes the ATP-regeneration economical. Moreover, such a polyphosphoric acid compound comprises linearly connected phosphate residues and therefore, it is heat-resistant, is not easily decomposed even if it is added to a subject to be inspected having a temperature ranging from about 60 to 70°C and thus it makes the measurement stable. Furthermore, the compound is not easily decomposed even by the action of the enzymes present in microorganisms since the phosphate residues are linearly connected together in the compound.

The polyphosphoric acid compound used in the present invention may be one derived from bacteria. Examples of such polyphosphoric acid compounds (n) are those having a value of n ranging from 10 to 1000. The polyphosphoric acid compound comprises 10 to 1000 phosphate residues linearly linked together and therefore, the compound is excellent in the heat resistance and resistance to bacteria.

Examples of polyphosphoric acid compounds usable in the present invention further include those biosynthesized by acting a polyphosphoric acid synthetase on ATP. The yield of the polyphosphoric acid compound can be improved to thus reduce the production cost thereof.

Any conventional method for preparing polyphosphoric acid compounds such as that disclosed in, for instance, J.P. KOKAI No. Hei 5-153993 may be used for the biosynthesis of the polyphosphoric acid compounds. This method comprises the step of acting a polyphosphoric acid synthetase on ATP and a polyphosphoric acid compound (n) in the presence of metal ions such as magnesium ions for the deactivation of the enzyme to thus biosynthesize a polyphosphoric acid compound (n+1).

The polyphosphoric acid synthetase used in the present invention has such characteristic properties that it acts on ADP and a polyphosphoric acid to thus form ATP and that it acts on ATP to give ADP and a polyphosphoric acid if ATP is present in excess, while ADP is absence.

The first ATP-regeneration reaction system of the present invention requires the presence of a trace amount of ATP and includes the step of converting ADP into ATP. Therefore, the polyphosphoric acid synthetase used herein is preferably free of any impurity, in particular, ATP and/or ADP. The polyphosphoric acid synthetase commonly available and derived from microorganisms in general comprises ATP and ADP and therefore, it is preferred to purify the commercially available product to thus reduce the content of ATP and/or ADP as low as possible prior to practical use thereof. In particular, if the first ATP-regeneration reaction system of the present invention is applied to the method for inspecting adenine nucleotide by the bioluminescence technique according to the present invention, the higher the content of ATP and/or ADP present in the polyphosphoric acid synthetase, the lower the accuracy of this inspection method. For this reason, the polyphosphoric acid synthetase preferably has an ATP concentration of not more than 10 pM, more preferably not more than 1 pM and most preferably 0 pM. On the other hand, the synthetase preferably has an ADP concentration of not more than 10 pM, more preferably not more than 1 pM and most preferably 0 pM.

In general, ADP is linked to the polyphosphoric acid synthetase in a rate of one molecule per 4 molecules of the latter and this becomes a principal cause of the contamination of the synthetase with ADP. For this reason, it is desirable to use a product obtained by mixing a polyphosphoric acid synthetase (10 to 100 μg/ml) with 0.1 mM of a polyphosphoric acid compound, removing the ADP by maintaining the mixture at a temperature of 37°C for 10 minutes to thus convert the ADP into ATP and adding a bioluminescence kit as will be detailed below to the mixture to thus purify the synthetase till any ATP is not detected any more.

Similarly, it is also preferred to use adenylate kinase, which is subjected to a purification treatment to reduce the concentrations of ATP and ADP to a level as low as possible. Accordingly, the adenylate kinase preferably has an ATP concentration of not more than 10 pM, more preferably not more than 1 pM and most preferably 0 pM. On the other hand, the adenylate kinase preferably has an ADP concentration of not more than 10 pM, more preferably not more than 1 pM and most preferably 0 pM.

The temperature of the first ATP-regeneration reaction system of the present invention usually ranges from 30 to 50°C and the time thereof suitably ranges from about 10 to 100 minutes. It is suitable in the first ATP-regeneration reaction system that the concentration of the adenylate kinase ranges from 1000 to 30000 Units (one unit is defined to be the activity of the enzyme required for generating one pM of ADP within one minute at 37°C), that the concentration of the polyphosphoric acid compound (n) ranges from 100 to 1000 μM and that the concentration of the polyphosphoric acid synthetase ranges from 100 to 10000 Units (one unit is defined to be the activity of the enzyme required for generating one pM of ATP within one minute at 37°C).

Then the second ATP-regeneration reaction system of the present invention will hereunder be described in detail.

The second ATP-regeneration reaction system of the present invention is one characterized in that it comprises the steps of acting a phosphotransferase on AMP in the presence of a polyphosphoric acid compound (n) (wherein n means the number of phosphate residues present therein) to convert them into ADP and a polyphosphoric acid compound (n-1) and then acting a polyphosphoric acid synthetase on the ADP in the presence of the polyphosphoric acid compound (n-1) to thus convert them into ATP and a polyphosphoric acid compound (n-2).

The polyphosphoric acid compound (n) used in the second ATP-regeneration reaction system is identical to that used in the first ATP-regeneration reaction system of the present invention.

It is likewise preferred, in the second ATP-regeneration reaction system of the present invention, to use a phosphotransferase and a polyphosphoric acid synthetase, which are purified to reduce the concentrations of ATP and ADP as low as possible. Therefore, the phosphotransferase preferably has an ATP concentration of not more than 10 pM, more preferably not more than 1 pM and most preferably 0 pM. In addition, the polyphosphoric acid synthetase used herein has the same ATP and ADP concentrations defined above.

The temperature of the second ATP-regeneration reaction system of the present invention usually ranges from 30 to 50°C and the time thereof suitably ranges from about 10 to 100 minutes. It is suitable in the second ATP-regeneration reaction system that the concentration of the polyphosphoric acid compound (n) ranges from 100 to 1000 μM, that the concentration of the AMP ranges from 10 to 1000 μM, that the concentration of the phosphotransferase ranges from 100 to 10000 Units (one unit is defined to be the activity of the enzyme required for generating one pM of ADP within one minute at 37°C) and that the concentration of the polyphosphoric acid synthetase ranges from 100 to 10000 Units (one unit is defined to be the activity of the enzyme required for generating one pM of ATP within one minute at 37°C).

Then a first embodiment of the method for inspecting adenine nucleotide according to the present invention will be detailed below.

The first embodiment of the method for inspecting adenine nucleotide according to the present invention comprises the steps of acting luciferase on ATP in the presence of luciferin and dissolved oxygen to thus generate AMP and to induce light emission and determining the quantity of emitted light, wherein the method is characterized in that it is provided with the first ATP-regeneration reaction system of the present invention.

The first embodiment of the method for inspecting adenine nucleotide according to the present invention can be expressed in terms of the following scheme:

(1) Light-Emission Reaction Using Luciferase:

$$\text{ATP} + \text{Luciferin} + \text{Dissolved Oxygen} \rightarrow \text{AMP} + \text{Oxyluciferin} + \text{Light Emission}$$

$$+ \text{Pyrophosphoric Acid} + CO_2$$

First ATP-Regeneration Reaction System
(2) Reaction Using Adenylate Kinase:

$$\text{Trace Amount of ATP} + \text{AMP} \rightarrow 2\text{ADP}$$

(3) Reaction Using Polyphosphoric Acid Synthetase:

$$2\text{ADP} + \text{Polyphosphoric acid compound (n)} \rightarrow 2\text{ATP} + \text{Polyphosphoric II}$$

$$\text{acid compound (n-2)}$$

In the foregoing scheme, if luciferase is acted on ATP, luciferin, dissolved oxygen and metal ions such as magnesium ions used for preventing deactivation of the enzyme, like the reaction (1), a reaction takes place, in which AMP, pyrophosphoric acid, oxyluciferin, carbon dioxide gas and light rays are generated and therefore, the determination of the light emitted by the action of this luciferase would permit the detection of ATP serving as an indicative component of contamination and in turn the judgment of the cleanliness factor in, for instance, food factories.

In this respect, if any ATP-regeneration reaction system is not present, the reaction (1) is terminated as the ATP is consumed and the luminescence disappears. For this reason, the method of the present invention employs the novel first ATP-regeneration reaction system.

This first ATP-regeneration reaction system includes the reaction (2), which comprises acting adenylate kinase on ATP and the AMP generated by the reaction (1) to thus convert them into 2 ADP molecules; and the reaction (3), which comprises the step of acting a polyphosphoric acid synthetase (PPK) on the foregoing 2 ADP molecules in the presence of metal ions such as magnesium ions used for preventing any deactivation of the enzyme and the polyphosphoric acid compound (n) to thus convert them into 2 ATP molecules and one molecule of polyphosphoric acid compound (n-2).

The essential points of these reactions will be described below. First of all, light rays are emitted and AMP is generated with the consumption of ATP according to the reaction (1). The AMP is converted into two ADP molecules through the reaction with the ATP remaining in the reaction system in the presence of the adenylate kinase (ADK) (the reaction (2)). The resulting two ADP molecules react with one molecule of the polyphosphoric acid compound (n) in the presence of the polyphosphoric acid synthetase (PPK). As a result, two phosphoric acid molecules are consumed during the reaction to thus give two ATP molecules and one molecule of polyphosphoric acid compound (n-2) (the reaction (3)). In this respect, it may likewise be conceivable that in this reaction, two ADP molecules react with two molecules of polyphosphoric acid compound (n) and two phosphoric acid molecules are consumed during the reaction to thus give two ATP molecules and two molecules of polyphosphoric acid compound (n-1). In this specification, the ATP-regeneration reaction system has been described on the basis of the former reaction scheme, but the present invention is not restricted to this reaction scheme.

Then the resulting ATP is employed and consumed in the reaction (1) to thus emit light rays and simultaneously, a part of the ATP is supplied to the reaction (2) in which it is employed in the conversion of AMP into ADP. Subsequently, the reaction (1) as an ATP-consumption system and the reactions (2) and (3) serving as an ATP-regeneration system are simultaneously and continuously conducted and repeated and the series of the reactions are sustained inasmuch as the polyphosphoric acid compound is present in the reaction system. In fact, the reactions are sustained till the number of phosphoric acid molecules n in the polyphosphoric acid compound (n) reaches about 3. In this respect, it is desirable to use a polyphosphoric acid compound (n) having the number n of not less than 10 or that comprising not less than 10 phosphoric acid residues linearly connected together, as a starting material for the polyphosphoric acid compound (n). For instance, if the reaction system contains a polyphosphoric acid compound having the number n ranging from about 40 to 80 in a concentration ranging from 200 to 300 $\mu$M, the resulting luminescence is stable over not less than 10 minutes and it has been confirmed that the luminescence is stable without causing any attenuation even after the elapse of not less than one hour. In this respect, if the number n is less than 9, a large amount of such compound should be added to the reaction system in order to sustain the luminescence over a long period of time.

The temperature used in the method for inspecting adenine nucleotide, which makes use of the first ATP-regeneration reaction system of the present invention, in general ranges from 30 to 50°C and the time required for the reaction system suitably ranges from 10 to 100 minutes. In this method, the luciferin and luciferase used may be a commercially available kit containing the same and the dissolved oxygen may be the oxygen present in the air. The concentrations of other reagents are the same as those used in the first ATP-regeneration reaction system.

The first method for inspecting adenine nucleotide according to the present invention is provided with the first ATP-regeneration reaction system of the present invention and therefore, the method permits not only an increase in the quantity of the bioluminescent light, but also the achievement of a substantially improved light-emission time.

In the first method for inspecting adenine nucleotide according to the present invention, the first ATP-regeneration reaction system can be preferably operated independently using a trace amount of ATP present in the subject to be inspected to thus amplify ATP in advance and a bioluminescent agent can then be added to the reaction system to substantially improve the sensitivity of the ATP-detection.

Incidentally, the first method for examining adenine nucleotide of the present invention has been described while taking the ATP-examination method by way of example, but the first method for examining adenine nucleotide is not restricted to this ATP-examination method but can likewise be applied to the examination of ADP. More specifically, the presence of ADP in the reaction system may trigger the first ATP-regeneration reaction system to thus give ATP as will be clear from the foregoing reaction scheme. However, it would be clear from the foregoing reaction scheme that this is not applicable to the AMP-examination. Accordingly, this can be applied to the determination of the degree of freshness of foods such as meat, raw fishes and vegetables.

Then a second embodiment of the method for inspecting adenine nucleotide according to the present invention will be detailed below.

The second embodiment of the method for inspecting adenine nucleotide comprises the steps of acting luciferase on ATP in the presence of luciferin and dissolved oxygen to thus generate AMP and induce light emission and determining the quantity of emitted light and the method is characterized in that it is provided with the second ATP-regeneration reaction system.

The second embodiment of the method for inspecting adenine nucleotide according to the present invention can be expressed in terms of the following scheme:

(1) Light-Emission Reaction Using Luciferase:

$$ATP + Luciferin + Dissolved\ Oxygen \rightarrow AMP + Oxyluciferin + Light\ Emission$$

$$+ Pyrophosphoric\ Acid + CO_2$$

Second ATP-Regeneration Reaction System
(2) Reaction Using Phosphotransferase:

$$AMP + Polyphosphoric\ acid\ compound\ (n) \rightarrow ADP + Polyphosphoric\ acid$$

$$compound\ (n-1)$$

(3) Reaction Using Polyphosphoric Acid Synthetase:

$$ADP + Polyphosphoric\ acid\ compound\ (n-1) \rightarrow ATP + Polyphosphoric\ acid$$

$$compound\ (n-2)$$

In the foregoing scheme, if luciferase is acted on ATP, luciferin, dissolved oxygen and metal ions such as magnesium ions used for preventing any deactivation of the enzyme, like the reaction (1), a reaction takes place, in which AMP, pyrophosphoric acid, oxyluciferin, carbon dioxide gas and light rays are generated and therefore, the determination of the light emitted by the action of the luciferase would permit the detection of ATP serving as an indicative component of contamination and in turn the judgment of the cleanliness factor in, for instance, food factories.

In this respect, if any ATP-regeneration reaction system is not present, the reaction (1) is terminated with the consumption of the ATP and the luminescence disappears. For this reason, the method of the present invention employs the novel second ATP-regeneration reaction system.

The second method for inspecting adenine nucleotide according to the present invention is provided with the second ATP-regeneration reaction system of the present invention and therefore, the method permits not only an increase in the quantity of the bioluminescent light, but also the achievement of a substantially improved light-emission time.

The essential points of these reactions will be described below. First of all, the ATP is consumed according to the reaction (1) and as a result, light rays are emitted and AMP is formed. The AMP is converted into ADP according to the reaction (2) and then the ADP is regenerated into ATP according to the reaction (3). Then this ATP is again supplied to the reaction (1) and light rays are generated with the consumption of the ATP. Subsequently, the reaction (1) serving as an ATP-consumption system and the reactions (2) and (3) serving as an ATP-regeneration system are simultaneously and continuously repeated. In this ATP-regeneration reaction system, AMP is converted into ATP through ADP without using any ATP derived from the contamination. Therefore, this system permits the achievement of an effect of sustaining the luminous time of the bioluminescence depending on the phosphate residue content of the polyphosphoric acid compound used, even at an extremely low ATP concentration and independent of the amount of ATP present in the system.

Incidentally, in the reaction (2) in which ADP is regenerated from the foregoing AMP, if a phosphotransferase (APP) is acted on the AMP in the presence of a polyphosphoric acid compound (n), one phosphate residue of the polyphosphoric acid compound is consumed to give ADP and a polyphosphoric acid compound (n-1). On the other hand, in the reaction (3) in which ATP is regenerated from the ADP, if a polyphosphoric acid synthetase (PPK) is acted on the ADP in the presence of the polyphosphoric acid compound (n-1), an additional phosphate residue of the polyphosphoric acid compound is further consumed to give ATP and a polyphosphoric acid compound (n-2).

The presence of the second ATP-regeneration reaction system of the present invention would permit the conversion of AMP into ATP through ADP in the regeneration reaction system in which AMP is converted into ATP, without using any ATP derived from the contamination and therefore, this system permits the achievement of an effect of increasing the amount of bioluminescence and of sustaining the luminous time of the bioluminescence depending on the phosphate residue content of the polyphosphoric acid compound used, even at an extremely low ATP concentration and independent of the amount of ATP present in the system. This in turn allows the improvement of the detection sensitivity of adenine nucleotide, the determination of the cleanliness factor and the determination of the degree of freshness of foods such as meat, raw fishes and vegetables, through the detection of invisible microorganisms in, for instance, foods factories. Moreover, this method permits the determination of AMP or ADP in addition to ATP serving as an indicative component of bacterial contamination. More specifically, the presence of AMP or ADP permits the operation of the second ATP-regeneration reaction system of the present invention and the formation of ATP as will be clear from the foregoing reaction scheme.

In the method for inspecting adenine nucleotide using the second ATP-regeneration reaction system of the present invention, the reaction temperature in general ranges from 30 to 50°C and the reaction time suitably ranges from about 10 to 100 minutes. In this method, the luciferin and luciferase used may be a commercially available kit containing the same and the dissolved oxygen may be the oxygen present in the air. The concentrations of other reagents are the same as those used in the second ATP-regeneration reaction system.

The second ATP-regeneration reaction system of the present invention permits more stable detection of adenine nucleotide present in a smaller amount whose detection using the first ATP-regeneration reaction system of the present invention is very difficult.

In other words, in the first ATP-regeneration reaction system of the present invention, two ADP molecules are formed using adenylate kinase (ADK) and a trace amount of ATP derived from bacterial contamination and whose content is to be determined, when AMP is converted into ADP according to the reaction (2). Then two ATP molecules are formed from the resulting two ADP molecules by the action of a polyphosphoric acid synthetase (PPK) in the reaction (3). Accordingly, in the first ATP-regeneration reaction system, the ATP derived from bacterial contamination and whose content is to be determined contributes to both the determination system (the reaction (1)) and the regeneration system (the reactions (2) and (3)). For this reason, the luminescent emission is quite stable and the luminescence sustains over a long period of time insofar as ATP is initially present in a sufficient amount. If it is intended to detect ATP present in an extremely low concentration, the initial reactions in the regeneration system (the reactions (2) and (3)) do not proceed successfully (the collision probability of a trace amount of ATP with a trace amount of ADP would be quite low and thus the reaction therebetween scarcely takes place), the luminescent emission is quite unstable and the luminescence may disappear within a very short period of time.

Contrary to this, the second ATP-regeneration reaction system of the present invention is so designed that the ATP-regeneration reaction can take place without using, in the regeneration system, any ATP derived from bacterial contamination so that an extremely trace amount of ATP can be detected. The second method for detecting adenine nucleotide according to the present invention is a method for examining adenine nucleotide on the basis of the bioluminescence, in which the novel second ATP-regeneration reaction system is incorporated.

Next, the method for detecting RNA through the bioluminescence according to the present invention will be detailed below.

The method for detecting RNA through the bioluminescence technique of the present invention comprises the steps of decomposing RNA present in a sample with an RNase to give mononucleotides, converting the AMP in the mononucleotides into ATP, acting luciferase on the ATP in the presence of luciferin and dissolved oxygen to induce light emission, determining the quantity of emitted light to thus quantitatively determine the amount of the AMP present in the RNA and the method is characterized in that it is provided with the second ATP-regeneration reaction system of the present invention.

The method for detecting RNA through the bioluminescence according to the present invention is provided with the second ATP-regeneration reaction system of the present invention and therefore, the process for converting the AMP among the nucleotides obtained from the RNA into ADP and then into ATP proceeds efficiently. Moreover, the AMP generated through the bioluminescence is again converted into ATP by the ATP-regeneration reaction system and as a result, the quantity of the light emitted by the bioluminescence can stably be sustained without causing any attenuation thereof and the AMP present in the RNA can be detected using an inexpensive luminometer having a simple structure.

In the present invention, the RNA present in a sample can be isolated according to any known method. An example of such a method for isolating RNA will hereunder be described, which comprises hydrolysis, centrifugation or electrophoresis or any combination thereof.

It is desirable to use centrifugation in order to isolate RNA from cells. For instance, the whole RNA can be extracted from yeast cells by treating the pulverized cells with phenol to thus break hydrogen bonds present in the macromolecules and to induce the modification of the proteins. The resulting opaque suspension is subjected to centrifugation to separate it into two phases. In this respect, the lower phenolic phase includes DNA, while the upper aqueous phase includes carbohydrates and RNA. Further the modified proteins are removed by centrifugation and then the RNA is precipitated by the addition of alcohol. At this stage, the resulting product is free of any DNA, but is contaminated with polysaccharides and therefore, the product may further be purified by treating with an amylase.

The RNA may be hydrolyzed into the constituent bases. More specifically, the RNA can be hydrolyzed into 5'-mononucleotides (such as AMP, GMP, CMP and UMP) by treating it with an RNase. Such an RNase usable herein may be any known one such as Nuclease S1.

Conventionally, the bases thus obtained are identified by, for instance, separating them by the paper chromatography and then detecting them using ultraviolet rays; or separating them by the electrophoresis (since each constituent base has a completely different charge in a citrate buffer solution having a pH value of 3.5) or separating them by a strongly acidic ion-exchange column chromatography and then identifying them using the ultraviolet absorption spectrum peculiar thereto.

In the present invention, RNA is hydrolyzed into the constituent bases with an RNase, the 5'-AMP out of the resulting nucleotides (5' AMP, GMP, CMP and UMP) is converted into ADP and then into ATP, the resulting ATP is subjected to the luciferin/luciferase reaction, the amount of the AMP picked up is estimated and thus the amount of the RNA is evaluated based on the estimated AMP level.

In the detection method of the present invention, which makes use of the second ATP-regeneration reaction system, the temperature in general ranges from 30 to 50°C and the time desirably ranges from about 10 to 100 minutes. In this method, the luciferin and luciferase used may be a commercially available kit containing the same and the dissolved oxygen may be the oxygen present in the air. For instance, the concentration of the RNase desirably ranges from 10 to 1000 Units (one unit is herein defined to be the enzyme activity required for making 1 μg of RNA soluble in an acid within one minute at 37°C) in case of Nuclease 51. The concentrations of other reagents are the same as those used in the second ATP-regeneration reaction system.

Then the method for amplifying ATP of the present invention, like a chain reaction, will be detailed below.

This method comprises the step of amplifying ATP like a chain reaction using the first ATP-regeneration reaction system. More specifically, the ATP-amplification method of the present invention is characterized by repeatedly conducting the following two steps: acting adenylate kinase on AMP in the presence of a trace amount of ATP to convert them into ADP and then acting a polyphosphoric acid synthetase on the resulting ADP in the presence of a polyphosphoric acid compound (n) (wherein n means the number of phosphate residues present therein) to convert them into ATP and a polyphosphoric acid compound (n-2).

First of all, in the first reaction stage of the reaction system, ATP and AMP immediately react with one another due to the action of the adenylate kinase to thus form two ADP molecules (first reaction). Then, the resulting two ADP molecules are reacted with a polyphosphoric acid compound (n) due to the action of a polyphosphoric acid synthetase to thus form two ATP molecules and a polyphosphoric acid compound (n-2) (second reaction). Thereafter, the reaction system proceeds to the second reaction stage of the reaction system and at this stage, the two ATP molecules generated in the first reaction stage of the system react with two molecules of AMP to thus form four ADP molecules (first reaction). Then, the resulting four ADP molecules are reacted with a polyphosphoric acid compound (n-2) to give four ATP molecules and a polyphosphoric acid compound (n-6) (second reaction). Subsequently, the reaction system is repeated several times such as third, fourth, fifth reaction stages and so on to thus increase the quantity of ATP. This reaction system is triggered by the addition of a trace amount of ATP to the first stage of the reaction system, then the reaction system proceeds like a chain reaction insofar as the polyphosphoric acid compound and AMP are present therein and thus the amount of ATP increases as a function of the power of two depending on the number of stages of the reaction system.

The temperature of the ATP-amplification method using the first ATP-regeneration reaction system of the present invention in general ranges from 30 to 50°C and the time required for the method suitably ranges from about 10 to 100 minutes. The AMP concentration required for the method suitably falls within the range of from about 10 to 100 µM. The concentrations of other reagents are the same as those used in the first ATP-regeneration reaction system.

The inventors of this invention have confirmed that reactions similar to the ATP-amplification reaction like a chain reaction take place even when creatine kinase and creatine phosphate or pyruvate kinase and phosphoenol pyruvic acid are used instead of the combination of a polyphosphoric acid compound and a polyphosphate kinase. In other words, it would be recognized that any combination of a phosphate compound with an enzyme might, in principle, be used for inducing a chain-like ATP-amplification reaction inasmuch as the combination permits the conversion of two ADP molecules into two ATP molecules. However, the polyphosphoric acid compound has an ability of synthesizing a large number of ATP molecules per molecule when combining the same with polyphosphate kinase and therefore, the use thereof is convenient in the ATP-amplification reaction which continuously takes place.

The temperature of the method for detecting adenine nucleotide characterized by the bioluminescence using luciferase after the amplification of ATP while making use of the first ATP-regeneration system of the present invention in general ranges from 30 to 50°C and the time required for the method suitably ranges from about 10 to 100 minutes. In this method, the luciferin and luciferase used may be a commercially available kit containing the same and the dissolved oxygen may be the oxygen present in the air. The concentrations of other reagents are the same as those used in the first ATP-regeneration reaction system.

When a trace amount of ATP is amplified by the method for amplifying ATP like a chain reaction according to the present invention, the resulting ATP is reacted with luciferase in the presence of luciferin and dissolved oxygen to form AMP and to induce light emission and the quantity of the generated light rays is determined, the quantity of light emitted, which corresponds to the increment of ATP, can be obtained and therefore, the method of the present invention permits the determination of such a trace amount of ATP, which has not conventionally been able to be detected. In principle, only one molecule of ATP present in the first reaction system can be detected by the method of the present invention.

Examples

The present invention will hereunder be described in more detail with reference to the following Examples and Comparative Examples.

Example 1

A bioluminescent system (present invention) for examining adenine nucleotide, which was provided with the first ATP-regeneration reaction system of the present invention and a bioluminescent system (Comparative Example) free of any ATP-regeneration reaction system were inspected for any change of emitted light with time under the following conditions:

(I) In Case of the Bioluminescent System Comprising the Incorporated First ATP-regeneration Reaction System (Example 1)

| | |
|---|---|
| (i) Polyphosphoric Acid Synthetase (PPK) | 1000 U (units) |
| (ii) Adenylate Kinase (ADK) | 22000 U (units) |
| (iii) Polyphosphoric acid compound (n = 65) | 260 µM (concentration) |
| (iv) ATP | 1.65 µM (concentration) |
| Subtotal: | 25 µl (volume) |
| (v) Biolumine Essence Kit | 25 µl (volume) |
| (available from Boehringer Mannheim Company) | |
| Total: | 50 µl (volume) |

The foregoing reagents (i) to (iii) required for the ATP-regeneration reaction system are admixed with the ATP (iv) serving as a subject to be inspected in such a manner that the total volume of the resulting mixture was equal to 25 µl. Then the mixture was reacted with 25 µl of Biolumine Essence Kit (v) (a bioluminescent agent mainly comprising luciferin and luciferase) and the quantity of light generated was determined using Luminometer (available from Amersham Pharmacia Biotech Company) to thus examine any change of the emitted light with time. The determination of the quantity of emitted light was conducted over 100 minutes after the initiation of the reaction at intervals of 10 minutes.

(II) In Case of the Bioluminescent System Free of Any Incorporated ATP-regeneration Reaction System (Comparative Example 1)

The same procedures used in Example 1 were repeated except that the foregoing reagent (ii) was omitted so that any ATP-regeneration reaction did not take place.

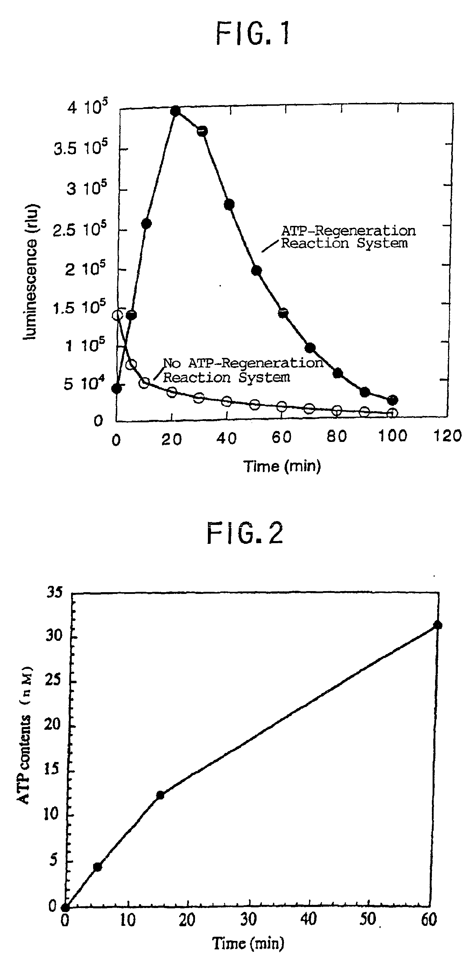

The results obtained in the foregoing bioluminescent systems (I) and (II) are plotted on Fig. 1 attached hereto. These results clearly indicate that in case of Comparative Example 1 free of any ATP-regeneration reaction system, the quantity of emitted light reaches a peak level at the initial stage of the reaction, it is reduced to a level of 1/3 time the peak value after 10 minutes from the initiation of the reaction and thereafter the quantity is gradually reduced as the elapse of time. Therefore, it would be concluded that the conventional method for determining adenine nucleotide shown in this Comparative Example requires the use of a precise luminometer. On the other hand, it is found, in case of Example 1 of the present invention, which makes use of an ATP-regeneration reaction system, that the quantity of emitted light is increased even to a level of 10 times that observed at the initial stage of the reaction after 20 minutes from the initiation of the reaction and that it is stable over a long period of time (not less than 30 minutes) without causing any attenuation, while it is maintained at such a high level. Accordingly, if a precise luminometer is used, the system of the present invention would permit the detection of even a trace amount of ATP and this in turn permits the considerable improvement of the accuracy of the food tests and hygienic examination. On the other hand, ATP can be detected using an inexpensive and simple luminometer in the food tests and hygienic examination without using any expensive and highly precise luminometer when it is sufficient to detect ATP at the conventional precision level.

Although a commercially available polyphosphoric acid synthetase is used in this Example without any pre-treatment, this commercially available enzyme includes ATP and ADP as impurities. Therefore, the ATP and ADP included in the enzyme as impurities may serve as a trigger of the foregoing ATP-regeneration reaction system even when the sample does not contain any ATP and/or ADP at all and in this case, luminescence is observed due to the presence of ATP and/or ADP as impurities even in the case where the sample is free of any ATP and/or ADP. For this reason, it is very important to reduce the adenine nucleotide concentration in the enzyme used to a level as low as possible in order to improve the accuracy of the measurement.

Example 2

This Example was conducted to make clear how the polyphosphoric acid synthetase (PPK) and phosphotransferase (APP) contribute in the ATP-regeneration reaction of the second ATP-regeneration reaction system of the present invention.

Materials Used in This Example

| | |
|---|---|
| (i) Buffer for Polyphosphoric Acid Synthetase (PPK buffer) | 3 µl |
| (ii) 0.1 mM (concentration) Adenisine Monophosphate (AMP) | 3 µl |
| (iii) 30 mM (concentration) Polyphosphoric acid compound (n = 65) | 3 µl |
| (iv) Ion-Exchanged Water | 19 µl |
| (v) Polyphosphoric Acid Synthetase (PPK) | 1 µl |
| (vi) Phosphotransferase (APP) | 1 µl |
| Total: | 30 µl (volume) |

PPK buffer: A mixture of (a) 50 mM HEPES buffer (pH 7.0), (b) 40 mM $(NH_4)_2SO_4$, and (c) $MgCl_2$.

The foregoing materials (i) to (vi) were incubated at a temperature of 30°C and samples (10 µl each) were taken from the mixture after 5, 15 and 60 minutes from the initiation of the reaction. Then the whole amount of each sample was added to a well for measurement to which 10 µl of luciferin and 5 µl of luciferase had been added in advance and the quantity of light emitted was determined using a luminometer (available from ARVO Company).

The ATP concentration of the sample at each sampling time can be estimated from the quantity of light emitted. The results thus obtained are plotted on Fig. 2. As will be seen from the data plotted on Fig. 2, the ATP concentration increases in the order 5 minutes < 15 minutes < 60 minutes after the initiation of the reaction, or as the time elapses. Thus, these experiments dearly indicate that ATP can be produced from AMP due to the action of the foregoing two enzymes or polyphosphoric acid synthetase (PPK) and the phosphotransferase (APP). Therefore, it would be recognized that these reactions can be used as an ATP-regeneration reaction system and that AMP can be detected by the bioluminescence while making use of this ATP-regeneration reaction system.

Example 3

A bioluminescent system (present invention) for examining adenine nucleotide, which was provided with the second ATP-regeneration reaction system of the present invention and a bioluminescent system (Comparative Example) free of any ATP-regeneration reaction system were inspected for any change of emitted light with time under the following conditions:

(I) In Case of the Bioluminescent System Comprising the Incorporated Second ATP- regeneration Reaction System (Example 3)

| | |
|---|---|
| (i) Buffer for Polyphosphoric Acid Synthetase (PPK buffer) | 3 µl |
| (ii) 0.1 mM (concentration) Adenosine Monophosphate (AMP) | 3 µl |
| (iii) 30 mM (concentration) Polyphosphoric acid compound (n = 65) | 3 µl |
| (iv) Ion-Exchanged Water | 19 µl |
| (v) Polyphosphoric Acid Synthetase (PPK) | 1 µl |
| (vi) Phosphotransferase (APP) | 1 µl |
| Total: | 30 µl (volume) |

(II) In Case of the Bioluminescent System Free of Any Incorporated Second ATP-regeneration Reaction System (Control; Comparative Example 3)

| | |
|---|---|
| (vii) 1.65 µM (concentration) Adenosine Triphosphate (ATP) | 3 µl |
| (viii) Ion-Exchange Water | 27 µl |
| Total: | 30 µl (volume) |

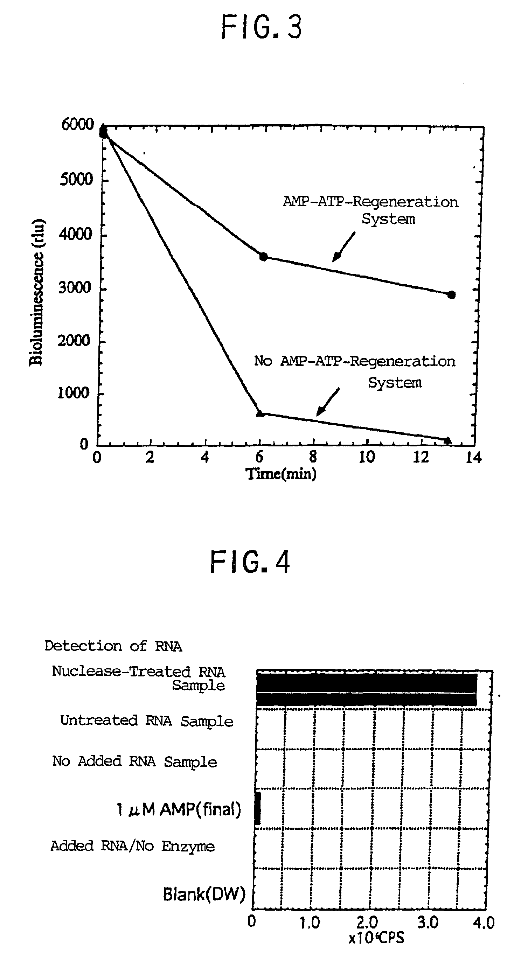

The foregoing materials (i) to (vi) for the sample comprising the ATP-regeneration reaction system and the foregoing materials (vii) to (viii) for the sample free of any ATP-regeneration reaction system were incubated at 30°C for 20 minutes, respectively. Then the whole amount of each sample was added to a well for measurement to which 10µl of luciferin and 5 µl of luciferase had been added in advance and the quantity of light emitted was determined using a luminometer (available from ARVO Company) to examine the changes of emitted light with time. The determination of the emitted light was conducted over 14 minutes after the initiation of the reaction or the determination of the change of the emitted light with time was carried out by measuring the quantity of the light emitted immediately after the initiation of the reaction and after 6 and 13 minutes from the initiation.
The results thus obtained are plotted on Fig. 3. The results thus obtained indicate that the quantity of the emitted light reached its peak level immediately after the initiation of the reaction, it was reduced to a level of not more than 1/6 time the peak level after 6 minutes from the initiation and subsequently, the quantity of the emitted light gradually reduced with time, in case of the bioluminescent system free of any ATP-regeneration reaction system. Therefore, it is clear that the conventional method requires the use of a precise luminometer. Contrary to this, it was found that the quantity of the emitted light reached its peak level immediately after the initiation of the reaction, it was not attenuated even after 6 minutes from the initiation and was stably maintained at a high level, in case of the bioluminescent system provided with the ATP-regeneration reaction system utilizing a polyphosphoric acid. Therefore, if a precise luminometer is used, the present invention permits the detection of even a trace amount of ATP and the improvement in the precision of the food tests and the hygienic examination. On the other hand, in the food tests and the hygienic examination where it is sufficient to achieve the conventional ATP-detection level, such an ATP-detection level can be achieved through the use of an inexpensive and simple luminometer rather than the use of an expensive highly precise luminometer.

Moreover, even when it is tried to detect an extremely dilute ATP, for instance, on the order of not more than 100 nM, the ATP-regeneration reaction system is stably operated irrespective of the initial ATP concentration and thus permits the detection of adenine nucleotide at a high sensitivity.

Example 4

(I) Isolation of RNA from Yeast

Reagent and Tools Used

| | |
|---|---|
| 1. Dry Yeast: | 200 g |
| 2. A Solution of Phenol (900 g/l): | 1 L |
| 3. Potassium Acetate (200 g/l; pH 5): | 200 mg |
| 4. Pure Ethanol: | 3 L |
| 5. Diethyl Ether: | 500 mg |
| 6. Five Thermostatic Chambers Maintained at 37°C | |

Methodology

Dry yeast (30 g) was suspended in 120 ml of water, which had been warmed at 37°C in advance. The suspension was maintained at that temperature for 15 minutes and 160 ml of a concentrated phenol solution was added thereto. After the suspension was mechanically stirred at room temperature for 30 minutes, it was subjected to centrifugation at 3000G for 15 minutes in a cold place to break the emulsion. The upper aqueous phase was collected using a Komagome pipette with caution, the aqueous phase was centrifuged in a cooled centrifuge at 10000G for 5 minutes to precipitate modified proteins. Potassium acetate was added to the resulting supernatant to a final concentration of 20 g/l and two volumes of ethanol were then added to precipitate the RNA present therein. The solution was ice-cooled, allowed to stand for one hour and then centrifuged at 2000G in a cold place for 5 minutes to collect the resulting precipitates. The resulting RNA was washed with an aqueous ethanol solution, ethanol and finally ether and dried in the air to thus isolate the RNA in an amount of about 4% of the dry mass of the yeast.

(II) Formation of Nucleotide by Hydrolysis of RNA

Reagents and Tools Used

| | |
|---|---|
| 1. Commercially Available tRNA | 2 μl |
| 2. Buffer Solution Attached to the Commercially Available tRNA (buffer) | 1 μl |
| 3. Distilled Water | 6.5 μl |
| 4. Nuclease S1 | 0.5 μl |

Methodology

The commercially available tRNA (2 μl) was dissolved in a mixture of 1 μl of the buffer attached to the tRNA, 6.5μl of distilled water and 0.5 μl of Nuclease S1 and the resulting solution was incubated at 37°C for 10 minutes. The AMP among the resulting 5'-mononucleotides was converted into ATP according to the following reactions and the amount of the ATP was determined by the bioluminescent technique.

(III) Identification of ATP by Bioluminescent Technique

Reagents

| 1. Buffer for Polyphosphoric Acid Synthetase (PPK buffer) | 3 µl |
| 2. 0.1 mM (concentration) Adenosine Monophosphate (AMP) | 3 µl |
| 3. 30 mM (concentration) Polyphosphoric acid compound (n = 65) | 3 µl |
| 4. Ion-Exchanged Water | 19 µl |
| 5. Polyphosphoric Acid Synthetase (PPK) | 1 µl |
| 6. Phosphotransferase (APP) | 1 µl |

Methodology

The foregoing reagents 1 to 6 were incubated at a temperature of 30°C, the whole solution was added to a well for measurement to which 10 µl of luciferin and 5 µl of luciferase had been added in advance after reacting them for 5, 15 and 60 minutes and the quantity of emitted light was determined using a luminometer (available from ARVO Company).

The ATP concentration can be obtained form the quantities of the emitted light thus determined after the reaction for a given time. The results are shown in Fig. 2. The figure shows that the ATP concentration increases with time. This experiment shows that the two enzymes, polyphosphoric acid synthetase (PPK) and phosphotransferase (APP) act on AMP to form ATP; accordingly, these reactions can be used as an ATP conversion system; and that AMP can be detected by bioluminescence to identify AMP which constitutes RNA

Example 5

Detection of RNA by Bioluminescent Technique

Reagents

| 1. Buffer for Polyphosphoric Acid Synthetase (PPK buffer) | 4 µl |
| 2. RNA Hydrolysate Obtained in Example 4 | 2 µl |
| 3. 30 mM (concentration) Polyphosphoric acid compound (n = 65) | 2 µl |
| 4. Ion-Exchanged Water | 7 µl |
| 5. Polyphosphoric Acid Synthetase (PPK) | 2 µl |
| 6. Phosphotransferase (APP) | 3 µl |

Methodology

The foregoing reagents 1 to 6 were incubated at a temperature of 30°C for 60 minutes, the whole solution was added to a well for measurement to which 10 µl of luciferin and 5 µl of luciferase had been added in advance and the quantity of emitted light was determined using a luminometer (available from ARVO Company).

The quantities of the emitted light thus determined are shown in the uppermost column on Fig. 4. As control samples, there were used a sample identical to that used above except that RNA free of any hydrolysis with nuclease was substituted for the reagent 2 (second column on Fig. 4) and a sample identical to that used above except that it was free of any added RNA and contained nuclease (third column on Fig. 4). The results thus obtained indicate that the decomposition of RNA is essential for the bioluminescence or that RNA must be decomposed to give AMP.

This is also clear from the fact that when pure AMP was indeed used as a sample, ATP was formed and bioluminescence was thus observed (fourth column on Fig. 4). In addition, the results observed for a sample to which RNA hydrolysate was added and which was free of any polyphosphoric acid synthetase and phosphotransferase are shown in the fifth column on Fig. 4. In this case, any ATP was not formed from AMP and therefore, the sample never caused any bioluminescence.

From the foregoing, it would be recognized that RNA could be detected by converting the AMP as an RNA-constituting nucleotide into ATP to thus induce bioluminescence. The sensitivity of the RNA-detection is dependent on the sensitivity of ATP-detection and therefore, the former is very high.

Example 6

In this Example, a bioluminescent system (present invention) for detecting adenine nucleotide provided with the first ATP-regeneration reaction system and a bioluminescent system (Comparative Example) free of any ATP-regeneration reaction system were inspected for the changes in emitted light with time under the following conditions, using a polyphosphoric acid synthetase derived from an enzyme and a polyphosphoric acid synthetase obtained by removing the ADP included therein as impurities through the purification of the foregoing synthetase:

(I) Process for Purifying Polyphosphoric Acid Synthetase (Preparation of PPK [1])

E. coli MV1184 cells (pBC10:ppk) were cultivated. Then the polyphosphoric acid synthetase was induced by the addition of 0.5 mM of IPTG after 2.5 hours from the initiation of the principal cultivation. After 2 hours, the resulting bacterial cells were collected and then suspended in 60 ml of Tris/HCl (+ sucrose) buffer. The suspension was frozen at -80°C and then thawed out on ice overnight. Then the cells were lysed by the addition of 250 µg/l of lysozyme. This was then sonicated, followed by the addition of 70 units/µl of DNase, 10 mg/l of RNase, removal of the unbroken bacterial cells through centrifugation, determination of the amount of the supernatant and addition of 0.2 g/l of ammonium sulfate to the supernatant. The enzyme was precipitated by centrifugation and dissolved by the addition of a phosphate buffer. The resulting solution was filtered through a 0.2 µm filter to remove the fine particles and then purified by hydrophobic chromatography (Phenyl Sepharose HP available from Pharmacia Company) and ion-exchange chromatography (MonoS5/5 available from Pharmacia Company). The resulting purified enzyme PPK [1] practically gave a single band on SDS/PAGE.

(II) Process for Removing ADP from Polyphosphoric Acid Synthetase PPK [1]

(Preparation of PPK [2])

The E. coli kinase PPK [1] prepared in the process (I) (200 µl ($5 \times 10^3$ units, 12 µg) was admixed with 0.1 mM of a polyphosphoric acid compound, the resulting mixture was maintained at 37°C for 10 minutes to convert the ADP linked to the enzyme into ATP and to thus liberate the same. To the mixture, there was added the purified polyphosphoric acid synthetase ($5 \times 10^5$ units) and the resulting mixture was maintained at 37°C for 10 minutes to remove the residual polyphosphoric acid compound (Any polyphosphoric acid synthetase is not eluted from the column if this step is omitted). The polyphosphoric acid synthetase thus eluted was purified by Smart System (MonoS available from Pharmacia Company) to give the purified enzyme PPK [2].
Any change in the emitted light with time was examined under the following conditions using the resulting purified enzymes PPK [1] and PPK [2]:

Example 6a: In Case Where Polyphosphoric Acid Synthetase PPK [1] was used

| | |
|---|---|
| (i) Polyphosphoric Acid Synthetase (PPK [1]) | 120 U (units) |
| (ii) Adenylate Kinase (ADK) | 90 U (units) |
| (iii) Polyphosphoric acid compound (n = 65) | 900 µM (concentration) |
| (iv) AMP | 600 µM (concentration) |
| (v) +ATP | 0.165 µM (concentration) |
| Subtotal: | 25 µl (volume) |
| (vi) Biolumine Essence Kit | 25 µl (volume) |
| (available from Boehringer Mannheim Company) | |
| Total: | 50 µl (volume) |

The materials (i) to (v) were mixed together and then the material (vi) was added to the resulting mixture after a desired reaction time to determine the quantity of emitted light.

Separately, the same procedures used above were repeated except for omitting the use of the reagent (v) and the quantity of emitted light was likewise determined.

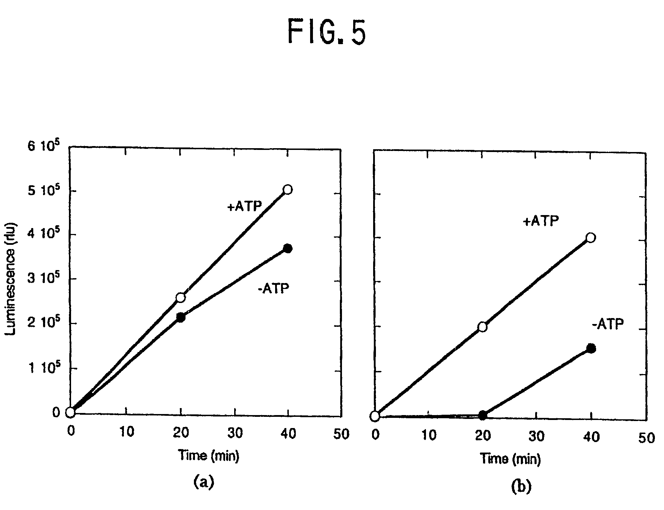

The results thus obtained are plotted on Fig. 5. Fig. 5(a) is a graph showing the results obtained by examining any influence of ATP (presence of added ATP: +ATP; free of any added ATP: -ATP) on the changes of the luminescence with time using a purified polyphosphoric acid synthetase (PPK [1]). Comparing these results, it is found that the intensity of emitted light observed when ATP is added (+ATP) is higher than that observed when ATP is not added.

Example 6b: In Case Where Polyphosphoric Acid Synthetase PPK [2] was used

The same procedures used in Example 6a were repeated except that the polyphosphoric acid synthetase PPK [2] in which the ADP had been removed was substituted for the polyphosphoric acid synthetase PPK [1]. The results thus obtained are plotted on Fig. 5b. Comparing the changes with time of the emitted light observed for the reaction system containing added ATP (+ATP) with those observed for the system free of any added ATP (-ATP), it is found that the intensity of emitted light observed when ATP is added (+ATP) is remarkably higher than that observed when ATP is not added.

The foregoing test results indicate that the ATP and ADP derived from the polyphosphoric acid synthetase serve as triggers in the ATP-regeneration reaction system of the present invention to thus induce luminescence and therefore, luminescence is observed even in the reaction system free of any added ATP (-ATP) in Example 6a and that the use of the polyphosphoric acid synthetase, which is purified to remove any ADP, would considerably reduce the rate of the ATP-amplification reaction and for this reason, the intensity of the emitted light observed in Example 6b (free of any added ATP (-ATP)) is substantially lower than that observed in Example 6a. Accordingly, it is concluded that the polyphosphoric acid synthetase should be sufficiently purified to ADP and ATP concentrations as low as possible prior to the practical use thereof in order to examine the presence of any ATP in a sample. The foregoing would permit the control of the ATP-amplification due to the presence of the ATP and ADP derived from an enzyme used to a lowest possible level and the highly precise examination of the presence of any ATP in a sample.

Example 7

In this Example, the concentrations of a polyphosphoric acid compound and AMP as substrates were changed using the purified polyphosphoric acid synthetase (PPK [2]) to thus determine any influence of the substrate concentration on the ATP-regeneration reaction system.

Example 7a

| | |
|---|---|
| (i) Polyphosphoric Acid Synthetase (PPK [2]) | 120 U (units) |
| (ii) Adenylate Kinase (ADK) | 90 U (units) |
| (iii) Polyphosphoric acid compound (n = 65) | 900 μM (concentration) |
| (iv) AMP | 600 μM (concentration) |
| (v) +ATP | 0.165 μM (concentration) |
| Subtotal: | 25 μl (volume) |
| (vi) Biolumine Essence Kit | 25 μl (volume) |
| (available from Boehringer Mannheim) | |
| Total: | 50 μl (volume) |

The materials (i) to (v) were mixed together and then the material (vi) was added to the resulting mixture after a desired reaction time to determine the quantity of emitted light.

Separately, the same procedures used above were repeated except for omitting the use of the reagent (v) and the quantity of emitted light was likewise determined.

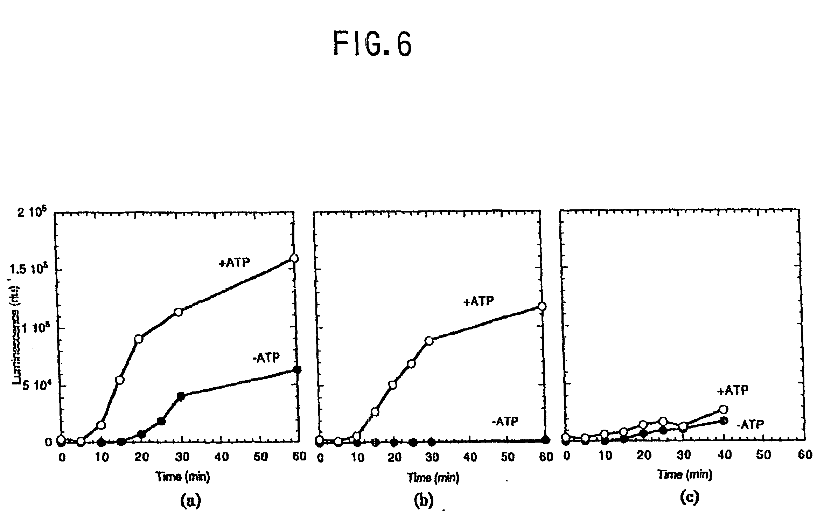

The results thus obtained are plotted on Fig. 6(a).

Example 7b

| (i) Polyphosphoric Acid Synthetase (PPK [2]) | 120 U (units) |
|---|---|
| (ii) Adenylate Kinase (ADK) | 90 U (units) |
| (iii) Polyphosphoric acid compound (n = 65) | 900 µM (concentration) |
| (iv) AMP | 60 µM (concentration) |
| (v) +ATP | 0.165 µM (concentration) |
| Subtotal: | 25 µl (volume) |
| (vi) Biolumine Essence Kit | 25 µl (volume) |
| (available from Boehringer Mannheim) | |
| Total: | 50 µl (volume) |

The same procedures used in Example 7a were repeated to determine the quantity of emitted light.
In addition, the same procedures used above were repeated except for omitting the use of the reagent (v) and the quantity of emitted light was likewise determined.
The results thus obtained are plotted on Fig. 6(b).

Example 7c

| (i) Polyphosphoric Acid Synthetase (PPK [2]) | 120 U (units) |
|---|---|
| (ii) Adenylate Kinase (ADK) | 90 U (units) |
| (iii) Polyphosphoric acid compound (n = 65) | 90 µM (concentration) |
| (iv) AMP | 600 µM (concentration) |
| (v) +ATP | 0.165 µM (concentration) |
| Subtotal: | 25 µl (volume) |
| (vi) Biolumine Essence Kit | 25 µl (volume) |
| (available from Boehringer Mannheim) | |
| Total: | 50 µl (volume) |

The same procedures used in Example 7a were repeated to determine the quantity of emitted light.
In addition, the same procedures used above were repeated except for omitting the use of the reagent (v) and the quantity of emitted light was likewise determined.
The results thus obtained are plotted on Fig. 6(c).
Referring to Fig. 6(a), it is found that if AMP and a polyphosphoric acid compound are present in sufficient amounts and an ATP-regeneration reaction system is present, the intensity of the emitted light is high and the luminescence lasts over a long period of time, while if any ATP-regeneration reaction system is not present, the intensity of the emitted light is considerably low. Referring now to Fig. 6(b), it is recognized that the AMP concentration is reduced to 1/10 time, the concentrations of the ADP and ATP present in the AMP as impurities are correspondingly reduced to 1/10 times, the luminescence due to the presence of the ADP or ATP derived from impurities (background) is accordingly controlled and the luminescence due to the added ATP becomes conspicuous. It would be quite noticeable that almost no ATP-amplification is observed and almost no light emission is likewise observed if any ATP is not added under such conditions. In other words, the background is almost zero. Accordingly, any trace amount of ATP present in a sample can be detected at a high precision.
Referring to Fig. 6(c), it is recognized that the intensity of the emitted light is low at a low concentration of the polyphosphoric acid compound even if AMP is present in a sufficient amount and an ATP-regeneration reaction system is present in the bioluminescent system, while the intensity of the emitted light is further reduced when any ATP-regeneration reaction system is not present. This clearly indicates that the concentrations of the AMP and polyphosphoric acid compound desirably range from about 10 to 10 μM and about 100 to 1000 μM, respectively, in order to ensure the sufficient functions of the second ATP-regeneration reaction system of the present invention.

The foregoing indicates that the adenine nucleotide-detecting sensitivity of the bioluminescent system provided with the ATP-regeneration reaction system can considerably be improved by reducing the concentrations of ADP and/or ATP present in an enzyme used in the ATP-regeneration reaction system of the present invention to a level as low as possible.

Example 8

To compare the conditions for the reaction free of any added ATP with those for the reaction in the presence of the added ATP, any change, with time, in emitted light was examined under the following conditions:

(I) Reaction Conducted in the Absence of Any Added ATP

| | |
|---|---|
| (i) Buffer Solution for Polyphosphoric Acid Synthetase | 10 μl |
| (ii) Adenosine Monophosphate (AMP) | 7.5 μl |
| (iii) Polyphosphoric acid compound (3mM, n = 65) | 22.5 μl |
| (iv) Polyphosphoric Acid Synthetase | 15 μl |
| (v) Adenylate Kinase | 3 μl |
| (vi) Distilled Water | 12 μl |
| Total: | 75 μl |

The foregoing reagents (i) to (vi) were admixed together, followed by the collection of samples (5 μl each) at predetermined intervals and the determination of the ATP concentration in each sample using an ATP-determination kit available from Boehringer Mannheim Company.

(II) Reaction Conducted in the Presence of Added ATP

| | |
|---|---|
| (i) Buffer Solution for Polyphosphoric Acid Synthetase | 10 μl |
| (ii) Adenosine Monophosphate (AMP) | 7.5 μl |
| (iii) Polyphosphoric acid compound (3mM, n = 65) | 22.5 μl |
| (iv) Polyphosphoric Acid Synthetase | 15 μl |
| (v) Adenylate Kinase | 3 μl |
| (vi) ATP (1.65 μM) | 5 μl |
| (vii) Distilled Water | 17 μl |
| Total: | 75 μl |

The foregoing reagents (i) to (vi) were admixed together, followed by the collection of samples (5 μl each) at predetermined intervals and the determination of the ATP concentration in each sample using an ATP-determination kit available from Boehringer Mannheim Company.

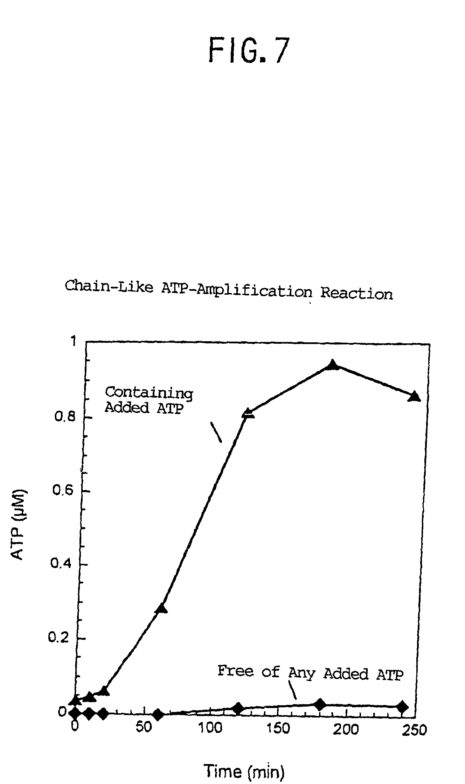

The results thus obtained are plotted on Fig. 7. The results shown in Fig. 7 indicate that the amount of ATP abruptly increased after 30 minutes from the initiation of the reaction and it reached its peak level after 180 minutes, in case where the reaction was conducted in the presence of the added ATP. Contrary to this, the amount of ATP never increased and was maintained at a low level, in case of the reaction free of any added ATP. This clearly indicates that the presence of a trace amount of added ATP may trigger the ATP-amplification reaction like a chain reaction.

The ATP-amplification method of the present invention permits the amplification of ATP present even in a trace amount and the highly sensitive detection thereof as well as the improvement of the precision of the food tests and hygienic examination. Moreover, the present invention likewise permits the detection of ATP with an inexpensive and simple luminometer.

Industrial Applicability

The first and second ATP-regeneration reaction systems of the present invention make use of a polyphosphoric acid compound having a large number of phosphate residues, for instance, not less than 10 of phosphate residues and a polyphosphoric acid synthetase. Accordingly, if these regeneration reaction systems are applied to the adenine nucleotide-detection method and/or RNA-detection method, which make use of the bioluminescence, the quantity of bioluminescent light can substantially be increased and the light emission time can likewise be considerably extended as compared with the conventional methods. As a result, the sensitivity of the adenine nucleotide detection or RNA-detection can be improved. Moreover, the use of the first ATP-regeneration reaction system of the present invention permits the efficient synthesis of ATP like a chain reaction at a low production cost.

**Claims**

1. An ATP-regeneration reaction system **characterized in that** it comprises the steps of acting adenylate kinase on AMP in the presence of a trace amount of ATP to convert them into two ADP molecules and then acting a polyphosphoric acid synthetase on the resulting two ADP molecules in the presence of a polyphosphoric acid compound (n) wherein n means the number of phosphate residues present in the polyphosphoric acid compound to convert them into two ATP molecules and a molecule of polyphosphoric acid compound (n-2).

2. An ATP-regeneration reaction system **characterized in that** it comprises the steps of acting a phosphotransferase on AMP in the presence of a polyphosphoric acid compound (n) wherein n means the number of phosphate residues present therein to convert them into ADP and a polyphosphoric acid compound (n-1) and then acting a polyphosphoric acid synthetase on the ADP in the presence of the polyphosphoric acid compound (n-1) to thus convert them into ATP and a polyphosphoric acid compound (n-2).

3. A method for detecting or inspecting adenine nucleotide according to the bioluminescence, comprising the steps of acting luciferase on ATP in the presence of luciferin and dissolved oxygen to thus generate AMP and induce light emission and determining the quantity of emitted light, wherein the method is provided with an ATP-regeneration reaction system **characterized in that** it comprises the steps of acting adenylate kinase on AMP in the presence of a trace amount of ATP to convert them into two ADP molecules and then acting a polyphosphoric acid synthetase on the resulting two ADP molecules in the presence of a polyphosphoric acid compound (n)(wherein n means the number of phosphate residues present therein to convert them into two ATP molecules and a molecule of polyphosphoric acid compound (n-2) and that the trace ATP is one derived from contamination.

4. A method for detecting or inspecting adenine nucleotide according to the bioluminescence, comprising the steps of acting luciferase on ATP in the presence of luciferin and dissolved oxygen to thus generate AMP and induce light emission and determining the quantity of emitted light, wherein the method is provided with an ATP-regeneration reaction system **characterized in that** it comprises the steps of acting a phosphotransferase on AMP in the presence of a polyphosphoric acid compound (n) wherein n means the number of phosphate residues present therein to convert them into ADP and a polyphosphoric acid compound (n-1) and then acting a polyphosphoric acid synthetase on the ADP in the presence of the polyphosphoric acid compound (n-1) to thus convert them into ATP and a polyphosphoric acid compound (n-2).

5. A method for detecting or inspecting adenine nucleotide according to the bioluminescence, comprising the steps of acting an RNase on RNA present in a sample to give mononucleotides, converting the AMP in the mononucleotides into ATP, acting luciferase on the ATP in the presence of luciferin and dissolved oxygen to induce light emission, and determining the quantity of emitted light to thus quantitatively determine the amount of the AMP present in the RNA, wherein the method is provided with an ATP-regeneration reaction system **characterized in that** it comprises the steps of acting a phosphotransferase on AMP in the presence of a polyphosphoric acid compound (n) wherein n means the number of phosphate residues present therein to convert them into ADP and a polyphosphoric acid compound (n-1) and then acting a polyphosphoric acid synthetase on the ADP in the presence of the polyphosphoric acid compound (n-1) to thus convert them into ATP and a polyphosphoric acid compound (n-2).

6. A method for amplifying ATP, like a chain reaction, **characterized in that** the method comprises repeatedly conducting the following two steps: acting adenylate kinase on AMP in the presence of a trace amount of ATP to convert them into two ADP molecules and then acting a polyphosphoric acid synthetase on the resulting two ADP molecules in the presence of a polyphosphoric acid compound (n) wherein n means the number of phosphate residues present therein to convert them into two ATP molecules and a molecule of polyphosphoric acid compound (n-2).

7. A method for detecting or inspecting adenine nucleotide, comprising the steps of acting luciferase on ATP in the presence of luciferin and dissolved oxygen to thus generate AMP and induce light emission and determining the quantity of emitted light, wherein the method comprises the steps of repeatedly conducting the following two steps: acting adenylate kinase on AMP in the presence of the ATP present in a subject to be tested to convert them into two ADP molecules and then acting a polyphosphoric acid synthetase on the resulting two ADP molecules in the presence of a polyphosphoric acid compound (n) wherein n means the number of phosphate residues present therein to convert them into two ATP molecules and a molecule of polyphosphoric acid compound (n-2) to thus amplify ATP like a chain reaction and then acting luciferase on the amplified ATP in the presence of luciferin and dissolved oxygen to thus give AMP and to induce light emission.

# FIG. 1

# FIG. 2

# FIG. 3

# FIG. 4

FIG. 5

(a)

(b)

EP 1 264 894 A1

## FIG.6

# FIG. 7

Chain-Like ATP-Amplification Reaction

### INTERNATIONAL SEARCH REPORT

| | |
|---|---|
| International application No. | PCT/JP01/00238 |

**A. CLASSIFICATION OF SUBJECT MATTER**
Int.Cl$^7$  C12P19/32, C12Q1/48, 1/66, 1/68

According to International Patent Classification (IPC) or to both national classification and IPC

**B. FIELDS SEARCHED**

Minimum documentation searched (classification system followed by classification symbols)
Int.Cl$^7$  C12P19/32, C12Q1/48, 1/66, 1/68

Documentation searched other than minimum documentation to the extent that such documents are included in the fields searched

Electronic data base consulted during the international search (name of data base and, where practicable, search terms used)
Medline,Biosis Previews

**C. DOCUMENTS CONSIDERED TO BE RELEVANT**

| Category* | Citation of document, with indication, where appropriate, of the relevant passages | Relevant to claim No. |
|---|---|---|
| X/A | WO, 98/48031, A (Yanasa Corporation), 1998-10-29 (Family: none) | 1,6/2-5,7 |
| PX/PA | SOL M.RESNICK et al., 'In Vitro ATP Regeneration from Polyphosphate and AMP by Polyphosphate:AMP Phosphotransferase and Adenylate Kinase from Acinetobacter johnsonii 210A.'Applied and Environmental Microbiology, May 2000, Vol.66, No.5, pp.2045-2051 | 2/1,3-7 |
| PA | T.SHIBA et al'Inorganic Polyphosphate and Polyphosphate Kinase:Their Novel Biological Functions and Application. 'Biochemistry (Moscow), 2000, Vol.65, No.3, pp.375-384 | 1-7 |
| A | EP, 794260, A (Kikkoman Corp.) 1997-09-10 & US, 5891659, A   & JP, 09-234099, A | 1-7 |
| A | T.NOGUCHI et al., 'Use of Escherichia coli Polyphosphate Kinase for Okigosaccharide Synthesis. 'Biosci. Biotechnol. Biochem., 1998, Vol.62, No.8, pp.1594-1596 | 1-7 |

☐ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 10 March, 2001 (10.03.01) | 21 March, 2001 (21.03.01) |

| Name and mailing address of the ISA/ | Authorized officer |
|---|---|
| Japanese Patent Office | |
| Facsimile No. | Telephone No. |

Form PCT/ISA/210 (second sheet) (July 1992)